# EUROPEAN PATENT SPECIFICATION

(11) **EP 0 588 372 B1**
(45) Date of publication and mention of the grant of the patent: **02.10.2002**
(21) Application number: 93116799.3
(22) Date of filing: 17.03.1989
(51) Int. Cl.: C07D 213/803

(54) **Process for preparing pyridine-2,3-dicarboxylic acid compounds**
Verfahren zur Herstellung von Pyridin-2,3-Dicarbonsäure-Verbindungen
Procédé de préparation de composés de l'acide pyridine-2,3-dicarboxylique

(30) Priority: 18.03.1988 JP 6677288
(43) Date of publication of application: 23.03.1994
(62) Divisional of application: 89903222.1
(73) Proprietor: SUGAI CHEMICAL INDUSTRY CO., LTD., Wakayama-shi, Wakayama 641 (JP)
(72) Inventor: Yamashita, Takaharu, c/o Sugai Chem. Ind. Co., Ltd, Wakayama 641 (JP); Kodama, Mitsuhiro, c/o Sugai Chem. Ind. Co., Ltd, Wakayama 641 (JP)
(74) Representative: Hansen, Bernd, Dr. Dipl.-Chem.

(56) References cited:
- EP-A- 0 220 518
- EP-A- 0 274 379
- JOURNAL OF THE CHEMICAL SOCIETY, vol. 438, part III, 1949, pages 2049-2054, The Chemical Society, London, GB; G.W. HOLTON et al.: "Experiments on the synthesis of rotenone and its derivatives. part XVI. The synthesis of abutic acid and its analogues"
- THE JOURNAL OF THE AMERICAN SOCIETY, vol. 72, September-December 1950, pages 5221-5225; L.H. CONOVER et al.: "Thiazole analogs of pyridoxine"

## Description

### Technical Field

This invention relates to a process for preparing pyridine-2,3-dicarboxylic acid compounds. More -particularly, it relates to a process for preparing pyridine-2,3-dicarboxylic acid compounds which are useful as an intermediate for manufacturing agricultural chemicals and pharmaceuticals.

### Background Art

Heretofore, as the method of preparing pyridine-2, 3-dicarboxylic acid compounds known are:
(1) Oxidation with nitric acid of quinolines and quinolinols which are synthesized by Skraup reaction from aniline and glycerine with concentrated sulfuric acid and nitrobenzene. (J. Chem. Soc. page 4433, 1956);
(2) Reacting an α,β-unsaturated hydrazone compound and a maleic acid compound in an inert solvent to obtain 1-(substituted amino)-1,4-dihydropyridine-2,3-dicarboxylic acid derivative. Then, heating the resultant derivative to eliminate the substituted amino group in the 1-position. (Japanese Patent Application Unexamined Publication No. 246369/1985).
(3) Treating a 1-(substituted amino)-1,2,3,4-tetrahydropyridine-2,3-dicarboxylic acid derivative with acid and/or by heat to convert into a 1,4-dihydropyridine-2,3-dicarboxylic acid derivative, and then oxidizing it. (Japanese Patent Application Unexamined Publication No. 47482/1986).
(4) Oxidizing quinoline with excess hypochlorites in the presence of ruthenium oxide. (Japanese Patent Application Unexamined Publication No. 212563/1986).
(5) Condensing and cyclizing α-halo-β-keto-esters and α,β-unsaturated aldehydes or ketones in the organic solvent in the presence of more than 2 mole equivalent of ammonium salt. (Japanese Patent Application Unexamined Publication No. 106081/1987).

As the method of synthesizing pyridinemonocarboxylic acid derivatives known is such a method as subjecting ethyl 2-methly-1,4-dihydronicotinate which is produced by condensation and cyclization of α,β-unsaturated aldehydes such as acrolein and crotonaldehyde with ethyl β-aminocrotonate to oxidation with nitric acid in a mixed acid. (J. Org. Chem. Soc. Vol. 21, page 800, 1956).

However, the above method (1) not only has many reaction processes but also requires drastic oxidation with nitric acid, and involves possible hazards. Also, the pyridine-2,3-dicarboxylic acids, which are apt to cause decarboxylation, result in low yields by the oxidation with nitric acid, and, in addition, produce a large quantity of acidic waste liquid. Thus, the method (1) is not suited to the industrial manufacture of pyridine-2,3-dicarboxylic acids.

The methods (2) and (3) mentioned above have many reaction processes leading to decreased total yields, and require the use of expensive starting materials. Particularly, they require elimination process of the substituted amino group in the intermediate. This decreases the yield and becomes a problem on resource saving. Therefore, it is difficult to manufacture pyridine-2,3-dicarboxylic acid derivatives industrially by the method (2) or (3).

In the method (4), there are problems that a large excess of the oxidant must be used and that a large quantity of waste liquid is produced requiring expenses for its disposal.

In the method (5), α-halo-β-keto-ester used as a raw material cannot be produced in good yield with conventional known manufacturing methods, causing high cost of raw material. It is, therefore, difficult to manufacture pyridine-2,3-dicarboxylic acid derivatives industrially by the method (5).

Accordingly, it is an object of this invention to provide a process for preparing pyridine-2,3-dicarboxylic acid compounds in high yield from starting materials inexpensive and readily available.

### Disclosure of Invention

A process for preparing pyridine-2,3-dicarboxylic acid compounds of the invention is a process for preparing pyridine-2,3-dicarboxylic acid compounds represented by the following formula (1). 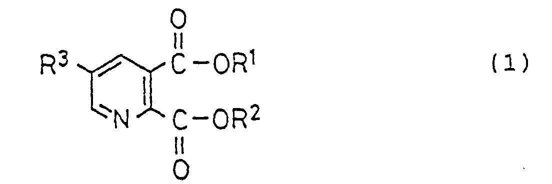 wherein R¹ and R² are, identical or different, a lower alkyl group, and R³ is a hydrogen atom or a lower alkyl group,
which comprises,
a compound of the following formula (5): 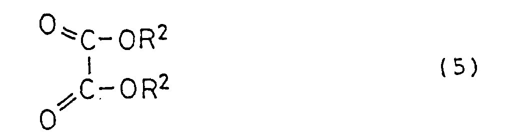 wherein R² is the same as defined above, is reacted with a compound of the following formula (6): 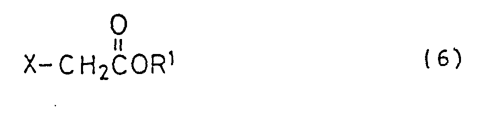 wherein R¹ and X are the same as defined above, in an aprotic solvent and in the presence of an alkali metal or a basic alkali metallic salt to give a compound of the following formula (7): 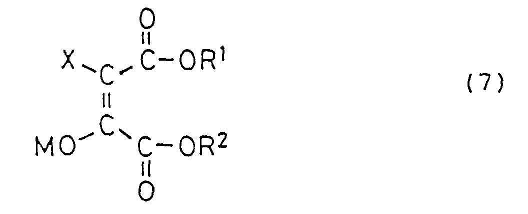 wherein R¹, R², X and M are the same as defined above,
then the compound of the above formula (7) is treated with an acid to give the compound of the following formula (3): 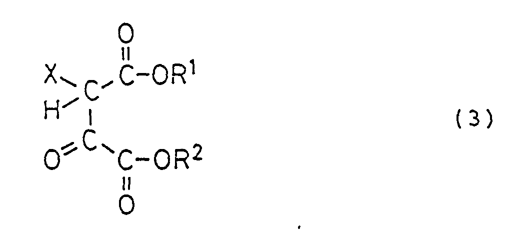 wherein R¹, R² and X are the same as defined above, then the compound of the above formula (3) and that of the following formula (4): 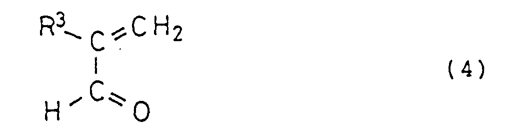 wherein R³ is the same as defined above, are reacted with ammonia.

In each formula mentioned above, examples of the lower alkyl groups for R¹, R², and R³ include, for example, straight or branched chain alkyl groups having 1 to 8 carbon atoms such as methyl, ethyl, propyl, isopropyl, butyl, isobutyl, tertiary-butyl, pentyl, hexyl, heptyl, octyl, 2-ethylhexyl group, and the like.

Examples of alkali metal for M include, for example, sodium, potassium, and the like.

Examples of the halogen atom for X include, for example, fluorine atom, chlorine atom, bromine-atom, and the like.

Examples of the compound of the formula (3) include, for example, dimethyl α-chlorooxalacetate, diethyl α-chlorooxalacetate, methyl ethyl α-chlorooxalacetate, dimethyl α-bromooxalacetate, diethyl α-bromooxalacetate, di-tertiary-butyl α-chlorooxalacetate, dipropyl α-chlorooxalacetate, dipropyl α-fluorooxalacetate, and the like.

Examples of the compound of the formula (4) include, for example, acrolein, 2-methyl-2-propenal, 2-ethyl-2-propenal, 2-propyl-2-propenal, 2-isopropyl-2-propenal, 2-butyl-2-propenal, 2-pentyl-2-propenal, 2-hexyl-2-propenal, 2-heptyl-2-propenal, 2-octyl-2-propenal, and the like.

Examples of the compound of the formula (5) include, for example, dimetyl oxalate, diethyl oxalate, dipropyl oxalate, diisopropyl oxalate, dibutyl oxalate, di-tertiary-butyl oxalate, dipentyl oxalate, dihexyl oxalate, diheptyl oxalate, dioctyl oxalate, and the like.

Examples of the compound of the formula (6) include, for example, methyl chloroacetate, methyl bromoacetate, methyl fluoroacetate, ethyl chloroacetate, ethyl bromoacetate, ethyl fluoroacetate, propyl chloroacetate, isopropyl chloroacetate, propyl bromoacetate, butyl chloroacetate, isobutyl chloroacetate, tertiary-butyl chloroacetate, butyl bromoacetate, hexyl chloroacetate, hexyl bromoacetate, octyl chloroacetate, and the like.

Examples of the compound of the formula (7) include, for example, dimethyl α-chlorooxalacetate sodium salt, diethyl α-chlorooxalacetate sodium salt, methyl ethyl α-chlorooxalacetate sodium salt, dimethyl α-bromooxalacetate potassium salt, diethyl α-bromooxalacetate potassium salt, di-tertiary-butyl α-chlorooxalacetate sodium salt, di-tertiary-butyl α-chlorooxalacetate potassium salt, dipropyl α-chlorooxalacetate sodium salt, dipropyl α-fluorooxalacetate potassium salt, and the like.

The preparation method of this invention can be shown with the following reaction scheme 1.

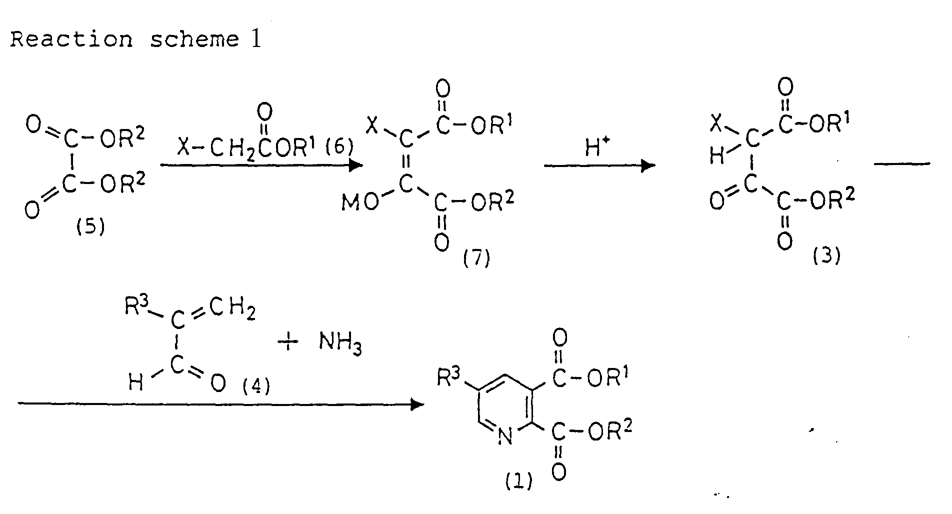

In the above formulae, R¹, R², R³, X, and M are the same as defined before.

The preparation process described in the reaction scheme 1 comprises a step which obtains the compound of the formula (7) by reacting the compound of the formula (5) and the compound of the formula (6) in an aprotic solvent and in the presence of an alkali metal or a basic alkali metallic salt (step 1), a step which obtains the compound of the formula (3) by treating the compound of the formula (7) prepared above with an acid (step 2), and a step which obtains the compound of the formula (1) by reacting the compound of the formula (3) prepared above, the compound of the formula (4) and ammonia (step 3).

In these steps 1 to 3, the compound of formula (7) obtained by step 1 and the compound of formula (3) obtained by step 2 can be used for the next steps without isolation or after isolation and purification by a conventional manner such as distillation etc.

The reaction of the above-mentioned step 1 is the reaction to condense the compounds of the formulae (5) and (6) in the aprotic solvent and in the presence of alkali metal or basic alkali metallic salt. Examples of the aprotic solvent used in the reaction include ethers such as dimethyl ether, diethyl ether, dibutyl ether, tetrahydrofuran, dioxane, ethylene glycol dimethyl ether, ethylene glycol diethyl ether, diethylene glycol dimethyl ether, and dibenzyl ether; aromatic hydrocarbons such as benzene, toluene, xylene, chlorobenzene, o-dichlorobenzene, and nitrobenzene, and the like.

Examples of alkali metal used include sodium and potassium, those of basic alkali metallic salt include alkali metallic alcoholates such as sodium methoxide, sodium ethoxide, and potassium tertiary-butoxide; alkyl alkali or aryl alkali such as methyl lithium, ethyl lithium, propyl lithium, n-butyl lithium, tertiary-butyl lithium, and phenyl lithium; alkaline metallic hydrides such as sodium hydride and potassium hydride, and the like.

The reaction usually is carried out at room temperature to elevated temperature at around 60°C and completed in about 10 hours to 3 days, usually in about 24 - 48 hours.

The compounds of the formulae (5) and (6) can be used at the proper molar ratio; for example, 0.8 - 2.0 mols, particularly 1.0 - 1.5 mols of the compound of the formula (6) to 1.0 mol of the compound of the formula (5) is used desirably. Alkali metal or basic alkali metallic salt is used at least an equimolar amount, desirably in excess, to the compound of the formula (5).

The compound of the formula (7) which is obtained in this way is used for the reaction of step 2 without isolation or after isolation and purification by a manner such as washing and recrystallizing.

The reaction of the step 2 is the reaction to obtain the compound of the formula (3) by treating the compound of the formula (7) with an acid. Any acids can be used for this reaction irrespective of inorganic or organic, and the acids exemplified in the step 1 of the reaction scheme 1 can be used.

The above-mentioned acid treatment is usually carried out by allowing the excess amount of acid to act on the compound of the formula (7) in the presence of water-insoluble solvent (e.g. diethyl ether, dibutyl ether, benzene, toluene, xylene, chlorobenzene, and the like) and water, and separating the water-insoluble solvent layer.

The compound of the formula (3) which is obtained in this way is used for the reaction of step 3 without isolation or after isolation and purification by a manner such as distillation and column chromatography.

The reaction of the step 3 is carried out to obtain the compound of the formula (1) by allowing the compound of the formula (3) obtained above to react with the compound of the formula (4) and ammonia.

The reaction is carried out under the condition free from solvent or in the organic solvent. Any organic solvent which does not affect the reaction can be used irrespective of polar, nonpolar, protic, or aprotic. Examples of solvents include alcohol such as methanol, ethanol, isopropyl alcohol, and butanol; halogenated hydrocarbons such as chloroform, 1,2-dichloroethane, and carbon tetrachloride; aromatic hydrocarbons such as benzene, toluene, xylene, chlorobenzene, o-dichlorobenzene, and nitrobenzene; ethers such as dimethyl ether, diethyl ether, dibutyl ether, tetrahydrofuran, dioxane, ethylene glycol dimethyl ether, ethylene glycol diethyl ether, diethylene glycol dimethyl ether, and dibenzyl ether; esters such as methyl acetate and ethyl acetate; aprotic polar solvents, for example, sulfoxides such as dimetyl sulfoxide, carboamides such as N,N-dimethylformamide, sulfones such as dimethyl sulfone and sulfolane, and hexamethylphosphoric triamide. The above-mentioned organic solvent can be used in a mixture of two or more types. Among them, aprotic organic solvents are desirable.

Reaction temperature is not particularly specified and the reaction can be carried out at various temperatures; for example, temperature range from 10 to 200°C; in particular, 35 - 130°C is more desirable. It is desirable to carry out the reaction under pressure, in particular, with ammonia pressurized at 0.3 - 2.5 kg/- cm². The reaction is completed in 30 minutes to 24 hours, in general in about 1 - 10 hours.

The compounds of the formulae (3) and (4) can be used at the proper molar ratio; for example, 0.8 - 1.5 mols, particularly 1.0 - 1.2 mols of the compound of the formula (4) to 1.0 mol of the compound of the formula (3) is used desirably. Ammonia is used usually in excess to the compounds of the formulae (3) and (4).

To increase the yield of the desired compound, the above-mentioned reaction is preferably carried out in the presence of ammonium salt such as ammonium carbonate, ammonium chloride, ammonium sulfate, ammonium nitrate, ammonium phosphate, and ammonium acetate.

The above-mentioned ammonium salt can be used in an adequate amount, but it is desirable to use 0.05 - 1.0 mol of the ammonium salt to 1 mol of the compound of the formula (4).

The ester compound of the formula (1) which is obtained through the process of this invention can be converted to corresponding pyridine-2,3-dicarboxylic acid compound by hydrolysis using the known method. The hydrolysis can be carried out in water or aqueous solvent by the conventional method using basic compounds such as alkali metal hydroxides including sodium hydroxide and potassium hydroxide; alkali metal carbonates such as sodium carbonate and potassium carbonate; and alkali metal hydrogen carbonates such as sodium hydrogencarbonate and potassium hydrogencarbonate, however, alkali metal hydroxides are desirable to achieve completely the reaction. The hydrolysis is carried out at room temperature to 150°C, preferably at 40 - 100°C and completed usually in about 1 - 24 hours.

The desired carboxylic acid compounds can be obtained by subjecting the carboxylic acid salts produced by the above hydrolysis to acid precipitation with a mineral acid such as hydrochloric acid, sulfuric acid, nitric acid, or phosphoric acid at 20 - 60°C.

In the aforementioned reaction scheme 1, the compounds of the formulae (5) and (6) which are starting materials are known compounds. As the process to prepare the compound of the formula (3), the method described in J. Amer., Chem., Soc., Vol. 72, 5221, (1950) is known, but the method described therein, namely the compound diethyl oxalacetate sodium salt is reacted with sulfuryl chloride or bromine in chloroform provides the object compound in the yield of as low as 31%, which is not suited to manufacturing the compound of the formula (3) in the industrial scale. According to the process of the invention, since the compound of the formula (5) is reacted with the compound of the formula (6), and then treating the resulting product with an acid, the compound of the formula (3) can be obtained in a high yield in contrast to the above method, resulting in improvement of the yield of the compound of the formula (1).

### Industrial Applicability

Pyridine-2,3-dicarboxylic acid compounds obtained by the process of this invention is useful as the intermediate for synthesizing various compounds such as agricultural chemicals and pharmaceuticals.

The process to prepare pyridine-2,3-dicarboxylic acid compounds according to the invention can produce the pyridine-2,3-dicarboxylic acid compounds with less steps and in high yield. In particular, it has a feature to carry out the reaction without isolating the intermediate. Further, the process permits the use of inexpensive readily available starting material, can be performed in safety because the reaction proceeds under the mild conditions, and realizes easy disposal of waste liquid. Thus, the pyridine-2,3-dicarboxylic acid compounds can be manufactured in the industrial scale.

### Examples

Hereinafter, the invention is described in detail with reference to Manufacturing example and Examples, but it should be understood that the invention is not limited to these examples alone.

### Manufacturing example 1

In a 300-ml four-neck flask were placed 41 g of dimethylamine hydrochloride (0.502 mol) and 42.7 g of 37% formalin (0.527 mol), and 84 g of n-decylaldehyde (0.527 mol) was added dropwise thereto at 20 to 30°C over a period of about 1 hour. After allowing to react for 6 hours at 70 to 75°C, the mixture was heated for 30 minutes at 110°C. Cooling down to room temperature, the solution was separated into oil layer and water layer. The oil layer (88 g) was distilled in vacuo, and 69 g of 2-(n-octyl)-2-propenal (bp₁₅: 108.5 to 110°C) was obtained.
IR (liquid film): 2930, 2850, 1700 cm⁻¹.

### Example 1

To 100 ml of diethyl ether, 5.8 g of metal sodium (0.252 mol) and 29.5 g of dimethyl oxalate (0.250 mol) were added, and 40 g of methyl chloroacetate (0.369 mol) was added dropwise over a period of 2 hours. After allowing to react for 48 hours at 25 to 30°C, the solution was diluted with 95 g of 35% acetic acid aqueous solution to be separated into ether layer and water layer. The water layer was further extracted with 100 ml of diethyl ether. The extract was mixed with the separated ether layer, and dried over anhydrous sodium sulfate. After distilling off diethyl ether, the residue was distilled in vacuo, and 22 g of dimethyl α-chlorooxalacetate (bp₈: 122°C) was obtained (yield 45.2%).

Next, in a 500-ml glass autoclave, 120 ml of toluene, 6.5 g of 2-ethyl-2-propenal (0.077 mol) and 10 g of the obtained dimethyl α-chlorooxalacetate (0.051 mol) were charged. After the internal temperature was raised to 90°C, bubbling of ammonia gas was started at ammonia pressure of 0.5 kg/cm², and the reaction was continued for 9 hours at 105°C. The content was cooled to room temperature, and the insoluble matter was filtered off. The filtrate was dried over anhydrous sodium sulfate, and the toluene was distilled off. The residue was distilled, and 6.6 g of 5-ethyl-2,3-dimethoxycarbonylpyridine (bp₄: 158.5 to 161°C) was obtained (yield 58.0%).
IR (liquid film) : 2950, 2850, 1740, 1720 cm⁻¹
Mass spectrometry : M⁺ = 223

### Example 2

To 250 ml of toluene, 5.8 g of metal sodium (0.252 mol) and 29.5 g of dimethyl oxalate (0.250 mol) were added, and 40 g of methyl chloroacetate (0.369 mol) was added dropwise at room temperature over a period of 1 hour. The reaction was continued for 48 hours at 45 to 55°C. To the reaction mixture, 60 ml of water and 35 g of 35% hydrochloric acid were added, and the mixture was separated into toluene layer and water layer. The toluene layer was dried over anhydrous sodium sulfate, and sodium sulfate was filtered off. This filtrate and 13.2 g of 2-ethyl-2-propenal (0.157 mol) were put in a 500 ml glass autoclave. After the internal temperature was raised to 90°C, bubbling of ammonia gas was started at ammonia pressure of 0.5 kg/ cm², and the reaction was continued for 4 hours at 105°C. The content was cooled to room temperature, the insoluble matter was filtered off. The filtrate was analyzed by gas chromatography, and 5-ethyl-2,3-dimethoxycarbonylpyridine was obtained in a yield of 30.6% based on the charged dimethyl oxalate.

### Example 3

To 250 ml of dibutyl ether, 5.8 g of metal sodium (0.252 mol) and 11.6 g of absolute ethanol (0.252 mol) were added, and the mixture was stirred for 1 hour. To the mixture, 36.8 g of diethyl oxalate (0.252 mol) and 33.0 g of ethyl chloroacetate (0.269 mol) were added dropwise at 30 to 35°C over a period of 1 hour. After the continuing the reaction for 38 hours at room temperature, 80 ml of water and 35 g of 35% hydrochloric acid were added to separate the solution into ether layer and water layer. The ether layer was dried over anhydrous sodium sulfate, and sodium sulfate was filtered off. This filtrate and 21.2 g of 2-ethyl-2-propenal (0.252 mol) were charged in a 500-ml glass autoclave. After the internal temperature was raised to 90°C, bubbling of ammonia gas was started at ammonia pressure of 0.5 kg/ cm², and the reaction was continued for 4 hours at 105°C. The content was cooled to room temperature, and the insoluble matter was filtered off. The filtrate was analyzed by gas chromatography, and 5-ethyl-2,3-diethoxycarbonylpyridine was obtained in a yield of 46.6% based on the charged diethyl oxalate.

## Claims

1. A process for preparing pyridine-2,3-dicarboxylic acid compounds represented by the formula (1): 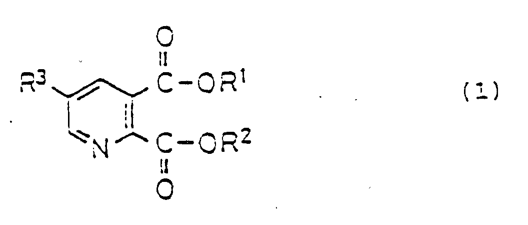 wherein R1 and R2 are, identical or different, a lower alkyl group, and R3 is a hydrogen atom or a lower alkyl group, which comprises reacting the compound of the formula (5): 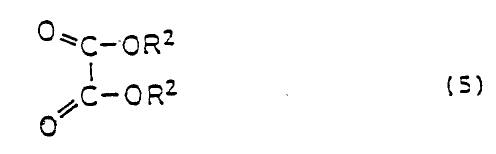 wherein R2 is the same as defined above, with the compound of the formula (6): 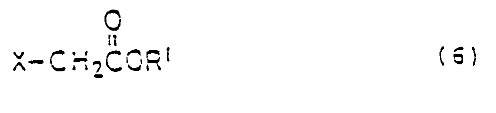 wherein R1 is the same as defined above and X is a halogen atom, in an aprotic solvent, and in the presence of an alkali metal or a basic alkali metallic salt to give the compound of the formula (7): 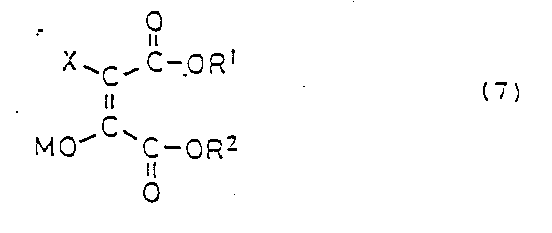 wherein R1, R2 and X are the same as defined above, and M is an alkali metal,
treating the resultant product with an acid to give the compound of the formula (3): 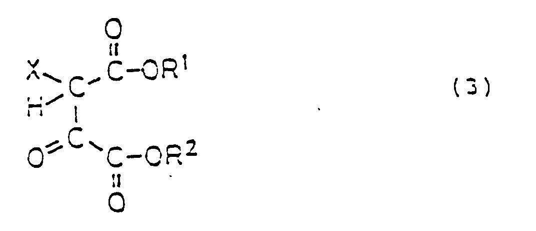 wherein R1, R2 and X are the same as defined above, and then reacting the resultant product with the compound of the formula (4): 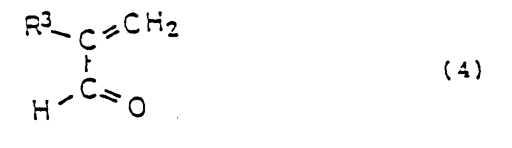 wherein R3 is the same as defined above, and ammonia.

2. A process for preparing pyridine-2,3-dicarboxylic acid compounds according to Claim 1, in which the reaction of the compound of the formula (5) with the compound of the formula (6) is carried out in ethers or aromatic hydrocarbons and in the presence of sodium metal, potassium metal, or alkali metallic alcoholate.

3. A process for preparing pyridine-2,3-dicarboxylic acid compounds according to Claim 1, in which the reaction of the compounds of the formulae (3) and (4) and ammonia is carried out under pressure and at a temperature of 10 - 200 °C.

4. A process for preparing pyridine-2,3-dicarboxylic acid compounds according to Claim 3, in which the reaction of the compounds of the formulae (3) and (4) and ammonia is carried out in the presence of an ammonium salt.

## Patentansprüche

1. Verfahren zur Herstellung von Pyridin-2,3-dicarbonsäure-Verbindungen dargestellt durch die Formel (1) : 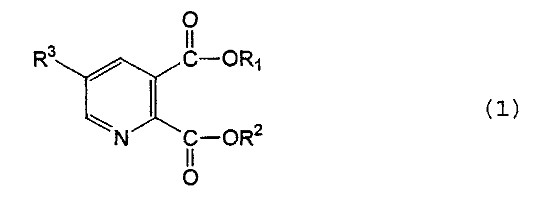 worin R¹ und R² gleich oder verschieden eine . Niederalkylgruppe sind und R³ ist ein Wasserstoffatom oder eine Niederalkylgruppe, umfassend das Reagieren der Verbindung mit der Formel (5): 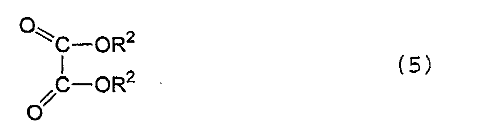 worin R² die gleiche Bedeutung wie oben definiert hat, mit der Verbindung der Formel (6): 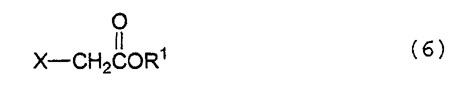 worin R¹ die gleiche Bedeutung wie oben definiert hat, und X ein Halogenatom ist in einem aprotischen Lösungsmittel und in Anwesenheit eines Alkalimetalls oder eines basischen Alkalimetallsalzes, um die Verbindung der Formel (7) zu ergeben: 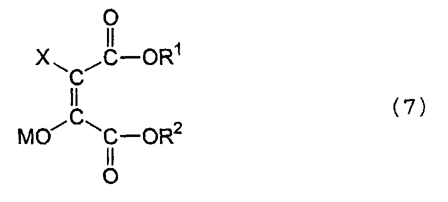 worin R¹, R² und X wie oben definiert sind, und M ein Alkalimetall ist;
Behandeln des erhaltenen Produkts mit einer Säure, um die Verbindung der Formel (3) zu ergeben: 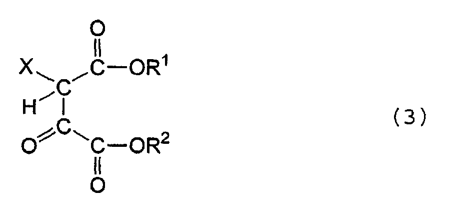 worin R¹, R² und X wie oben definiert sind, und anschließend Reagieren des erhaltenen Produkts mit der Verbindung der Formel (4): 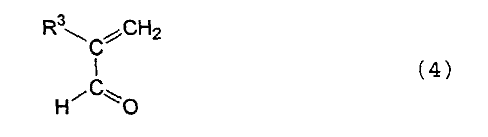 worin R³ die gleiche Bedeutung wie oben definiert hat, und Ammoniak.

2. Verfahren zur Herstellung von Pyridin-2,3-dicarbonsäure-Verbindungen gemäß Anspruch 1, bei dem die Reaktion der Verbindung der Formel (5) mit der Verbindung der Formel (6) in Ether oder aromatischen Kohlenwasserstoffen in Anwesenheit eines Natriummetalls, Kaliummetalls oder Alkalimetallalkoholaten durchgeführt wird.

3. Verfahren zur Herstellung von Pyridin-2,3-dicarbonsäure-Verbindungen gemäß Anspruch 1, bei dem die Reaktion der Verbindungen der Formeln (3) und (4) und Ammoniak unter Druck und bei einer Temperatur von 10 bis 200°C durchgeführt wird.

4. Verfahren zur Herstellung von Pyridin-2,3-dicarbonsäure-Verbindungen gemäß Anspruch 3, bei dem die Reaktion der Verbindungen der Formeln (3) und (4) und Ammoniak in Anwesenheit eines Ammoniumsalzes durchgeführt wird.

## Revendications

1. Procédé de préparation de dérivés de l'acide pyridine-2,3-dicarboxylique, représentés par la formule (1) : 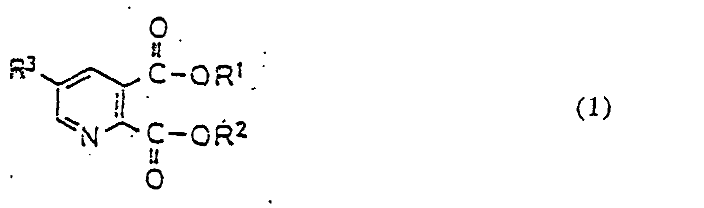 dans laquelle R¹ et R² sont identiques ou différents et représentent chacun un groupe alkyle inférieur et R³ représente un atome d'hydrogène ou un groupe alkyle inférieur, procédé qui comprend la réaction d'un composé de formule (5) : 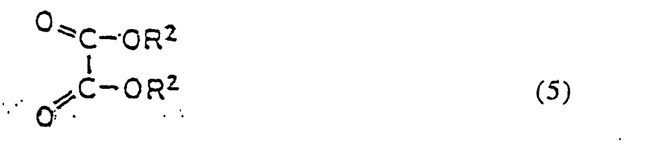 dans laquelle R² a la signification indiquée précédemment, avec un composé de formule (6) : 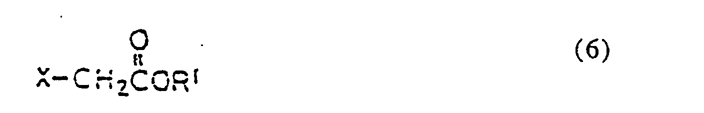 dans laquelle R¹ a la signification indiquée précédemment et X représente un atome d'halogène, dans un solvant aprotique, en présence d'un métal alcalin ou d'un sel basique d'un métal alcalin, pour obtenir un composé de formule (7) : 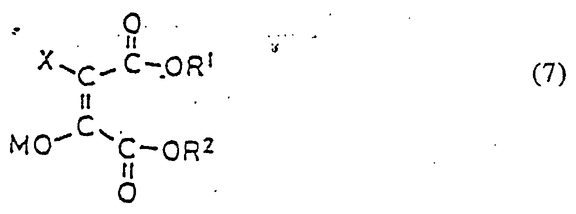 dans laquelle R¹, R² et X ont les significations indiquées précédemment, et M représente un métal alcalin,
le traitement du produit résultant avec un acide pour obtenir un composé de formule (3) : 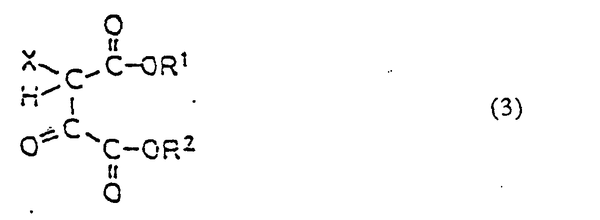 dans laquelle R¹, R² et X ont les significations indiquées précédemment,
et ensuite la réaction du produit résultant avec un composé de formule (4) : 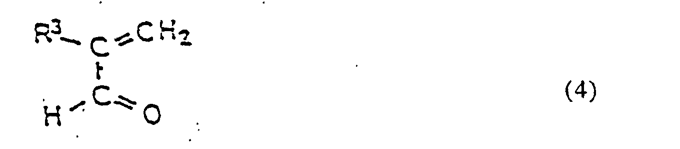 dans laquelle R³ a la signification indiquée précédemment, et de l'ammoniac.

2. Procédé de préparation de dérivés de l'acide pyridine-2,3-dicarboxylique selon la revendication 1, dans lequel on réalise la réaction du composé de formule (5) avec le composé de formule (6) dans un éther ou un hydrocarbure aromatique, en présence de sodium métallique, de potassium métallique ou d'un alcoholate de métal alcalin.

3. Procédé de préparation de dérivés de l'acide pyridine-2,3-dicarboxylique selon la revendication 1, dans lequel on réalise la réaction du composé de formule (3) avec le composé de formule (4) et l'ammoniac sous pression, à une température de 10 à 200°C.

4. Procédé de préparation de dérivés de l'acide pyridine-2,3-dicarboxylique selon la revendication 3, dans lequel on réalise la réaction du composé de formule (3) avec le composé de formule (4) et l'ammoniac en présence d'un sel d'ammonium.
